# EUROPÄISCHE PATENTSCHRIFT

(11) **EP 2 621 419 B1**
(45) Veröffentlichungstag und Bekanntmachung des Hinweises auf die Patenterteilung: **20.04.2016**
(21) Anmeldenummer: 11748404.8
(22) Anmeldetag: 24.08.2011
(51) Int. Cl.: B65D 1/32, B65D 23/08, A61F 9/00, A61M 11/02, A61M 15/08, A61M 35/00

(54) **TROPFENSPENDER MIT ZUSAMMENDRÜCKBAREM MEDIENBEHÄLTER**
DROP DISPENSER COMPRISING A COMPRESSIBLE MEDIA CONTAINER
DISTRIBUTEUR DE GOUTTES À RÉSERVOIR DE FLUIDE COMPRIMABLE

(30) Priorität: 28.09.2010 DE 102010047840
(43) Veröffentlichungstag der Anmeldung: 07.08.2013
(73) Patentinhaber: Aptar Radolfzell GmbH, 78315 Radolfzell (DE)
(72) Erfinder: GREINER-PERTH, Jürgen, 78244 Gottmadingen (DE); WOCHELE, Matthias, 78239 Rielasingen-Worblingen (DE)
(74) Vertreter: Patentanwaltskanzlei Cartagena
(86) Internationale Anmeldenummer: PCT/EP2011/064579
(87) Internationale Veröffentlichungsnummer: WO 2012/041614

(56) Entgegenhaltungen:
- EP-A1- 1 213 003
- EP-A1- 1 338 520
- DE-A1- 2 906 818
- DE-U1- 29 800 793
- FR-A1- 2 687 568
- GB-A- 780 922
- GB-A- 2 419 821
- US-A1- 2002 036 180
- US-A1- 2008 264 958

## Beschreibung

Die Erfindung betrifft einen Tropfenspender zur Ausbringung eines pharmazeutischen Mediums mit einem Medienbehälter, mit einer an einer Öffnung des Medienbehälters angebrachten Auslasseinheit mit einer Auslassöffnung und mit einer abnehmbaren Kappe, gemäß dem Oberbegriff des Anspruchs 1, wie aus EP 1 213 003 A1 bekannt. Dabei ist der Medienbehälter bei abgenommener Kappe frei zugänglich und quer zur Haupterstreckungsrichtung des Tropfenspenders elastisch verformbar, um dadurch eine Ausbringung von Medium durch die Auslassöffnung hindurch zu bewirken. Weiterhin ist die Kappe derart ausgebildet, dass sie im aufgesetzten Zustand die Auslassöffnung und/oder eine zum Druckausgleich vorgesehene Einlassöffnung an der Auslasseinheit mechanisch schützt und/oder flüssigkeitsdicht oder gasdicht gegenüber der Umgebung trennt.

Eine weitere derartige Austragvorrichtung ist aus der DE 102008001313 B3 bekannt. Diese weist eine Kappe auf, die im aufgesetzten Zustand einen sich an den Medienbehälter anschließenden Applikatorabschnitt überdeckt.

Ähnliche Austragvorrichtungen sind aus dem Stand der Technik allgemein bekannt. Ihre Medienbehälter werden üblicherweise als Quetschflaschen oder Sqeezebottles bezeichnet. Die Besonderheit liegt bei solchen Austragvorrichtungen darin, dass die Medienbehälter aus einem elastisch verformbaren Material, insbesondere einem elastisch verformbaren Kunststoff, gefertigt sind und bestimmungsgemäß zum Austrag des Mediums, insbesondere zum Austrag einer Flüssigkeit, radial zu ihrer Haupterstreckungsrichtung durch gleichzeitige beidseitige manuelle Kraftbeaufschlagung zusammengedrückt werden. Hierdurch wird der Druck innerhalb des Medienbehälters erhöht, so dass das Medium durch die Auslassöffnung hindurch ausgetragen wird.

Gattungsgemäße Tropfenspender verfügen aufgrund dieser Möglichkeit der Druckbeaufschlagung des Mediums mittels des Medienbehälters über keine hiervon separate Fördereinrichtung wie einer Kolbenpumpe. Sie sind daher vergleichsweise preisgünstig herzustellen.

Als nachteilig an solchen Austragvorrichtungen wird die Gefahr angesehen, diese versehentlich zu betätigen. So kann beispielsweise eine in einem Kulturbeutel, einer Toilettentasche oder einer Handtasche aufbewahrte Austragvorrichtung unbeabsichtigt durch die Handhabung der Tasche so zusammengedrückt werden, dass Flüssigkeit austritt. Diese Problematik wird bei solchen Austragvorrichtungen noch verschärft, die keine flüssigkeitsdichte Abdichtung der Auslassöffnung mittels der Kappe bewirken. Dies ist bei Austragvorrichtung gegeben, bei denen die Kappe primär dem mechanischen Schutz der Austragöffnung dient. Sie kann in einem solchen Fall beispielsweise Belüftungsöffnungen aufweisen, die bei einem unbeabsichtigten Betätigen der Austragvorrichtung das Austreten von Flüssigkeit ermöglichen.

Die beschriebene Problematik ergibt sich insbesondere bei Tropfenspendern, da bei diesen bereits durch geringe Kräfte ein Austragvorgang bewirkt werden kann, um eine wohldosierten Austrag von Tropfen zu ermöglichen.

Aus GB 2 419 821 A ist ein Inhaliergerät mit einer Tropferflasche bekannt, deren Tropferöffnung mittels einer sich über einen erheblichen Teil der Flaschenlänge erstreckenden Kappe verschließbar ist. Die Kappe ist Teil eines Gehäuses, das die nicht als komprimierbar beschriebene Flasche insgesamt umgibt.

GB 780 922 und US 2002/036180 A1 beschreiben formstabile Behälter mit sich bis zum Behälterboden erstreckenden Kappen.

### Aufgabe und Lösung

Aufgabe der Erfindung ist es, einen gattungsgemäßen Tropfenspender dahingehend weiterzubilden, dass ein unbeabsichtigtes Austreten von Medium aus dem Medienbehälter vermieden wird.

Erfindungsgemäß werden hierfür die kennzeichnenden Merkmale des Anspruchs 1 vorgesehen

Bei einem erfindungsgemäßen Tropfenspender ist demnach vorgesehen, dass die Kappe nicht nur einen Schutz der Auslassöffnung oder gegebenenfalls eines Einlassöffnung darstellt, sondern im aufgesetzten Zustand darüber hinaus den Medienbehälter vor einer unbeabsichtigten Kraftbeaufschlagung schützt und somit die unbeabsichtigte Ausbringung von Flüssigkeit aus dem Medienbehälter verhindert. Der Mantelfortsatz der Kappe erstreckt sich hierzu über mindestens die Hälfte der freien Länge des Medienbehälters, also derjenigen Länge zwischen dem Übergang der starren Auslasseinheit zum Medienbehälter und dem gegenüberliegenden Ende des Medienbehälters.

Durch den Mantelfortsatz werden radial zur Haupterstreckungsrichtung wirkende Kräfte aufgenommen, so dass diese keine Deformation des Medienbehälters bewirken können. Vorzugsweise ist der Mantelfortsatz der Kappe zur Aufnahme dieser Kräfte aus einem verformungssteiferen Material als der Medienbehälter ausgebildet und/oder gegenüber dem Medienbehälter dickwandiger ausgebildet.

Es hat sich gezeigt, dass die Überstreckung von mindestens 50% der Länge des Medienbehälters zwischen der Auslasseinheit und seinem entgegengesetzten Ende üblicherweise die Gefahr einer Betätigung bereits deutlich reduziert.

Dennoch wird es als vorteilhaft angesehen, wenn der Mantelfortsatz sich noch weiter bis in Richtung des Endes des Medienbehälters erstreckt. Bevorzugt wird eine Länge von mindestens 75% der Länge des Medienbehälters zwischen der Auslasseinheit und dem entgegengesetzten Ende des Medienbehälters, insbesondere vorzugsweise sogar von mindestens 90%. Auch sind Ausgestaltungen denkbar, bei denen der Mantelfortsatz sich sogar noch über das Ende des Medienbehälters hinaus erstreckt, so dass dieser bei aufgesetzter Kappe nicht mehr über das der Auslassöffnung entgegengesetzte Ende der Kappe hinausragt.

Besonders vorteilhaft ist die Kappe gemäß der vorgenannten Beschreibung bei Tropfenspendern, die zur Erzeugung eines nahezu überdrucklosen Austritts der Flüssigkeit durch die Auslassöffnung ausgebildet sind, so dass bereits eine geringfügige Deformation des Medienbehälters ausreichen kann, um einen für den Austritt erforderlichen Überdruck, beispielsweise einen zum Öffnen eines Auslassventils erforderlichen Überdruck, zu erzeugen. Vorzugsweise ist der Medienbehälter so ausgebildet, dass bereits eine Betätigungskraft von zwei Newton bei vollständig gefülltem Medienbehälter ausreicht, um einen Tropfen auszubringen.

Erfindungsgemäß ist der Mantelfortsatz ausreichend starr, um zu verhindern, dass eine beidseitig auf den Mantelfortsatz jeweils aufgebrachte manuelle Kraft von 20 Newton zu einer Verformung des Mantelfortsatzes führt, die eine Verformung des Medienbehälters zur Folge hätte. Kräfte jenseits dieser Grenze von etwa 20 Newton werden nur selten unbeabsichtigt aufgebracht. Es reicht daher üblicherweise aus, wenn die Kappe so starr ist, dass sie bei einer derartigen beidseitig auf die Kappe wirkenden Kraft die Komprimierung des Medienbehälters verhindert.

Bei einer besonders sicheren Gestaltung des Tropfenspenders erstreckt sich der Mantelfortsatz umlaufend über die genannte Länge von mindestens 50%, mindestens 75% oder mindestens 90%. Üblicherweise ist jedoch auch eine Ausgestaltung im Hinblick auf die Sicherheit ausreichend, bei der die Kappe mindestens eine, vorzugsweise mindestens zwei, umfänglich beabstandete und vorzugsweise einander gegenüberliegende Aussparungen im Mantelfortsatz aufweist. Bei einer solchen Gestaltung ragt der Mantelfortsatz somit zumindest an seinem der Auslassöffnung abgewandten Ende nicht mehr umlaufend in Richtung des distalen Endes des Medienbehälters, sondern weist voneinander beabstandete Zungen auf, zwischen denen die genannten Aussparungen angeordnet sind. Die genannten Aussparungen helfen beim Abziehen der Kappe, indem sie ein Festhalten des Medienbehälters zu diesem Zweck gestatten.

Um zu verhindern, dass eine ungünstige Kraftbeaufschlagung des Tropfenspenders im Bereich dieser Aussparung einer Kraftbeaufschlagung des Medienbehälters verursacht, wird es als bevorzugt angesehen, wenn die Aussparungen so geformt sind, dass sich über mindestens 50% der Länge des Medienbehälters zwischen der Auslasseinheit und dem entgegengesetzten Ende des Medienbehälters der Mantelfortsatz umlaufend geschlossen ist oder die jeweilige Breite der Aussparungen einen Winkel von 90° in Umfangsrichtung nicht übersteigt.

### Kurzbeschreibung der Zeichnungen

Weitere Aspekte und Merkmale der Erfindung ergeben sich außer aus den Ansprüchen auch aus der nachfolgenden Beschreibung eines bevorzugten Ausführungsbeispiels der Erfindung, welches anhand der Figuren nachfolgend erläutert wird. Dabei zeigen:
- Fig. 1: einen erfindungsgemäßen Tropfenspender, bei der eine Kappe von einem Hauptabschnitt mit Medienbehälter und Auslasseinheit getrennt ist,
- Fig. 2: den Tropfenspender der Fig. 1 bei aufgesetzter Kappe in einer Seitenansicht und
- Fig. 3: den Tropfenspender der Fig. 1 bei aufgesetzter Kappe in einer Ansicht von unten.

### Detaillierte Beschreibung des Ausführungsbeispiels

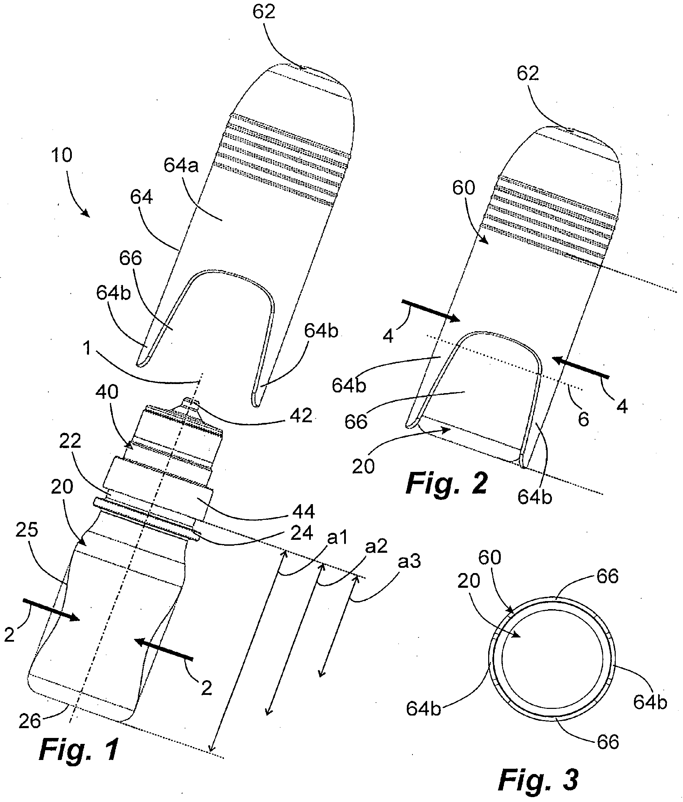

Ein Tropfenspender 10 der Fig. 1 weist drei separate Bauteile auf. Diese umfassen einen Medienbehälter 20, eine Auslasseinheit 40 sowie eine Kappe 60, wobei die Auslasseinheit auch aus mehreren, im vorliegenden Zusammenhang nicht weiter differenzierten Bauteilen bestehen kann. Die Auslasseinheit 40 verfügt über eine Auslassöffnung 42 und ist aus einem gegenüber dem Medienbehälter vergleichsweise starren Kunststoff hergestellt. Sie ist über eine nicht dargestellte Schnappverbindung mit einem Hals 22 des Medienbehälters 20 verbunden.

Der Medienbehälter 20 besteht aus einem dünnwandigen, elastisch leicht verformbaren Kunststoffmaterial. Er dient der Aufnahme von Flüssigkeit, insbesondere einer Augentropfenflüssigkeit. Zum Austragen der Flüssigkeit durch die Auslassöffnung 42 ist vorgesehen, dass der Medienbehälter 20 bei geringem Kraftaufwand in Richtung der Pfeile 2 und wie durch die gestrichelte Linie 25 angedeutet zusammengedrückt wird, wobei hierdurch das Innenvolumen des Medienbehälters 20 reduziert wird und durch den damit erzeugten Druck Flüssigkeit durch die Auslassöffnung 42 hindurch ausgetragen wird. Dabei ist im Bereich der Auslassöffnung 42 in nicht näher dargestellter Art und Weise ein druckabhängig öffnendes Auslassventil vorgesehen, welches bereits bei sehr geringem Überdruck öffnet, um einen Austragvorgang bei ebenfalls sehr geringem Überdruck zu ermöglichen, der zur Erzeugung von Tropfen im Bereich der Auslassöffnung 42 geeignet ist.

Zum Schutz der Auslassöffnung ist die bereits genannte Kappe 60 vorgesehen. Diese Kappe 60 ist in nicht näher dargestellter Art und Weise dafür ausgebildet, im aufgesetzten Zustand über zwei umlaufende Ringabschnitte 24, 44 der Auslasseinheit 40 und des Medienbehälters 20 gestützt zu werden. Die Kappe 60 schützt die Auslassöffnung 42 insbesondere mechanisch. Selbst im aufgesetzten Zustand der Kappe 60 bleiben jedoch eine oder mehrere Durchbrechungen 62 in der Kappe offen, um ein Trocknen der Umgebung der Auslassöffnung 42 zu gestatten.

Die Kappe 60 erfüllt darüber hinaus eine weitere Funktion: Im aufgesetzten Zustand der Kappe unterbindet die Kappe 60 wirksam ein Zusammendrücken des Medienbehälters 20. Sie weist hierzu einen mantelförmigen Fortsatz 64 auf, der sich im aufgesetzten Zustand der Kappe 60, dargestellt in Fig. 2, bis zu dem der Auslassöffnung 42 gegenüberliegenden Boden 26 des Medienbehälters 20 erstreckt. Da die Kappe 60 aus einem vergleichsweise starren Material gefertigt ist, führt eine unbeabsichtigte Kraftbeaufschlagung der Kappe 60 in Richtung der Pfeile 4 nicht zu einem Zusammendrücken des Medienbehälters 20 und somit nicht zu einem Austragvorgang, welcher aufgrund der Öffnungen 62 in der Kappe unmittelbar ein Austreten der Flüssigkeit in eine Umgebung bewirken könnte.

Bei der vorliegenden Gestaltung wird eine freie Länge a1 des Medienbehälters 20 zwischen dem zum Boden 26 weisenden Ende der Auslasseinheit 40 und dem Boden 26 vollständig vom Mantelfortsatz 64 überspannt. Dies wird als ideal angesehen. Da der Medienbehälter 20 durch seinen Boden 26 jedoch dort eine erhöhte Eigensteifigkeit aufweist, wäre gegebenenfalls auch ein Mantelfortsatz ausreichend, der mindestens eine Länge a2 oder zumindest eine Länge a3 überspannt, die 75% bzw. 50% der freien Länge a1 des Medienbehälters 20 entspricht.

Um ein leichtes Abnehmen der Kappe 60 trotz des vollständigen Überspannens des Medienbehälters 20 zu gewährleisten, weist der Mantelfortsatz 64 zwei gegenüberliegende Aussparungen 66 auf, die ein Ergreifen des Medienbehälters 20 zum Zwecke des Abziehens der Kappe 60 gestatten. In Umfangsrichtung zwischen diesen Aussparungen 66 erstrecken sich Zungen 64b des Fortsatzes 64 bis zum Boden 26 des Medienbehälters 20. Um trotz dieser zwei Aussparungen 66 zu gewährleisten, dass eine unbeabsichtigte Betätigung nicht stattfinden kann, sind diese Aussparungen 66 nur in einem unteren Abschnitt des mantelförmigen Fortsatzes 64 vorgesehen. Durch den umlaufend geschlossenen Abschnitt 64a des mantelförmigen Fortsatzes 66 und durch die Tatsache, dass zumindest bis zu der gepunktet dargestellten Linie 6 die zwei Aussparungen jeweils in Umfangsrichtung kleiner 90° sind, wird die Wahrscheinlichkeit eines unbeabsichtigten Austragvorgangs gering gehalten.

Der dargestellte und beschriebene Tropfenspender 10 ist bereits durch geringe Kraftbeaufschlagung des Medienbehälters 20 in Richtung der Pfeile 20 zu betätigen, so dass eine wohldosierte Flüssigkeitsabgabe möglich ist. Durch die Kappe 60 und deren Mantelfortsatz 64 ist gewährleistet, dass nach Aufsetzen der Kappe eine unbeabsichtigte Betätigung nicht oder kaum stattfinden kann.

## Patentansprüche

1. Tropfenspender (10) zur Ausbringung eines pharmazeutischen Mediums, insbesondere einer pharmazeutischen Flüssigkeit, wobei die Austragvorrichtung insbesondere als Ophthalmik-Tropfenspender ausgestaltet ist, mit
- einem als Quetschflasche ausgebildeten Medienbehälter (20),
- einer an einer Öffnung des Medienbehälters angebrachten Auslasseinheit (40) mit einer Auslassöffnung (42) und
- einer abnehmbaren Kappe (60),
wobei
- der Medienbehälter (20) bei abgenommener Kappe (60) frei zugänglich und quer (2) zu seiner Haupterstreckungsrichtung (1) elastisch verformbar ist, um eine Druckbeaufschlagung des Mediums im Medienbehälter (20) und dadurch eine Ausbringung von Medium durch die Auslassöffnung (42) hindurch zu bewirken, und
- die Kappe (60) im aufgesetzten Zustand die Auslassöffnung (42) und/oder eine zum Druckausgleich vorgesehene Einlassöffnung an der Auslasseinheit (40) mechanisch schützt und/oder flüssigkeitsdicht oder gasdicht gegenüber der Umgebung trennt, wobei
die Kappe (60) einen Mantelfortsatz (64) aufweist, der sich bis in den Bereich des Medienbehälters (20) erstreckt und diesen zumindest abschnittsweise umgibt **dadurch gekennzeichnet, dass** der Mantelfortsatz sich bezogen auf eine Aufsetzrichtung (1) er Kappe über mindestens 50% der Länge (a1) des Medienbehälters (20) zwischen der Auslasseinheit (40) und einem entgegengesetzten Ende (26) des Medienbehälters (20) erstreckt und wobei der Mantelfortsatz (64) ausreichend starr ist, um zu verhindern, dass eine beidseitig auf den Mantelfortsatz (64) aufgebrachte manuelle Kraft von jeweils 20N zu einer Verformung des Mantelfortsatzes (64) führt, die eine Verformung des Medienbehälters (20) und dadurch einen Austragvorgang zur Folge hat.

2. Tropfenspender nach Anspruch 1,
**dadurch gekennzeichnet, dass**
sich der Mantelfortsatz (64) bezogen auf die Aufsetzrichtung (1) der Kappe (60) über mindestens 75% der Länge (a1) des Medienbehälters (20) zwischen der Auslasseinheit (40) und dem entgegengesetzten Ende (26) des Medienbehälters (20) erstreckt.

3. Tropfenspender nach Anspruch 2,
**dadurch gekennzeichnet, dass**
sich der Mantelfortsatz (64) bezogen auf die Aufsetzrichtung (1) der Kappe (60) über mindestens 90% der Länge (a1) des Medienbehälters (20) zwischen der Auslasseinheit (40) und dem entgegengesetzten Ende (26) des Medienbehälters (20) erstreckt.

4. Tropfenspender nach einem der vorstehenden Ansprüche,
**dadurch gekennzeichnet, dass**
die Kappe (60) mindestens eine, vorzugsweise mindestens zwei, umfänglich beabstandete und vorzugsweise einander gegenüberliegende Aussparungen (66) im Mantelfortsatz (64) aufweist.

5. Tropfenspender nach Anspruch 4,
**dadurch gekennzeichnet, dass**
die Aussparungen (66) so geformt sind, dass über mindestens 50% der Länge (a1) des Medienbehälters (20) zwischen der Auslasseinheit (40) und dem entgegengesetzten Ende (26) des Medienbehälters (20) der Mantelfortsatz (64) umlaufend geschlossen ist oder die jeweilige Breite der Aussparungen (66) in Umfangsrichtung einen Winkel von 90° nicht übersteigt.

## Claims

1. Drop dispenser (10) for dispensing a pharmaceutical medium, in particular a pharmaceutical liquid, wherein the dispensing device is configured, in particular, in the form of an ophthalmic drop dispenser, having
- a media container (20), designed in the form of a squeeze bottle,
- an outlet unit (40), which is fitted on an opening of the media container and has an outlet opening (42), and
- a removable cap (60),
wherein
- the media container (20), with the cap (60) removed, is freely accessible and can be elastically deformed in a direction transverse (2) to the main direction of extent (1) thereof in order to cause the medium in the media container (20) to be subjected to pressure and thus medium to be dispensed through the outlet opening (42), and
- the cap (60), once placed in position, mechanically protects the outlet opening (42) and/or an inlet opening provided for pressureequalizing purposes on the outlet unit (40), and/or separates the same in a liquid-tight or gas-tight manner in relation to the surroundings,
wherein the cap (60) has a lateral-surface extension (64), which extends into the region of the media container (20) and encloses the latter at least in part,
**characterized in that** the lateral-surface extension, as seen in relation to a placing-in-position direction (1) of the cap, extends over at least 50% of the length (a1) of the media container (20) between the outlet unit (40) and an opposite end (26) of the media container (20), and wherein the lateral-surface extension (64) is sufficiently rigid to prevent a manual force of 20N applied to either side of the lateral-surface extension (64) resulting in deformation of the lateral-surface extension (64), said deformation resulting in deformation of the media container (20) and thus in a dispensing operation.

2. Drop dispenser according to Claim 1,
**characterized in that**
the lateral-surface extension (64), as seen in relation to the placing-in-position direction (1) of the cap (60), extends over at least 75% of the length (a1) of the media container (20) between the outlet unit (40) and the opposite end (26) of the media container (20).

3. Drop dispenser according to Claim 2,
**characterized in that**
the lateral-surface extension (64), as seen in relation to the placing-in-position direction (1) of the cap (60), extends over at least 90% of the length (a1) of the media container (20) between the outlet unit (40) and the opposite end (26) of the media container (20).

4. Drop dispenser according to one of the preceding claims,
**characterized in that**
the cap (60) has at least one cutout (66) in the lateral-surface extension (64), preferably at least two cutouts spaced apart along the circumference and preferably located opposite one another.

5. Drop dispenser according to Claim 4,
**characterized in that**
the cutouts (66) are formed such that the lateral-surface extension (64) is closed all the way round over at least 50% of the length (a1) of the media container (20) between the outlet unit (40) and the opposite end (26) of the media container (20), or the respective width of the cutouts (66) in the circumferential direction does not exceed an angle of 90°.

## Revendications

1. Distributeur de gouttes (10) pour l'expulsion d'un fluide pharmaceutique, en particulier d'un liquide pharmaceutique, dans lequel le dispositif de délivrance est constitué en particulier par un distributeur de gouttes ophtalmiques, avec
- un réservoir de fluide (20) sous forme de flacon comprimable,
- une unité de sortie (40) avec une ouverture de sortie (42) placée sur une ouverture du réservoir de fluide, et
- un capuchon amovible (60),
dans lequel
- lorsque le capuchon (60) est enlevé, le réservoir de fluide (20) est librement accessible et est élastiquement déformable transversalement (2) à sa direction d'extension principale (1), afin d'exercer une pression sur le fluide dans le réservoir de fluide (20) et de provoquer ainsi une expulsion de fluide à travers l'ouverture de sortie (42), et
- lorsqu'il est mis en place, le capuchon (60) protège mécaniquement l'ouverture de sortie (42) et/ou une ouverture d'entrée prévue pour l'équilibrage de pression sur l'unité de sortie (40) et/ou la sépare de l'ambiance de façon étanche au liquide ou étanche au gaz,
dans lequel le capuchon (60) présente un prolongement d'enveloppe (64), qui s'étend jusque dans la région du réservoir de fluide (20) et entoure celui-ci au moins localement,
**caractérisé en ce que** le prolongement d'enveloppe s'étend, par rapport à une direction (1) de mise en place du capuchon, sur au moins 50 % de la longueur (a1) du réservoir de fluide (20) entre l'unité de sortie (40) et une extrémité opposée (26) du réservoir de fluide (20) et dans lequel le prolongement d'enveloppe (64) est suffisamment rigide pour empêcher qu'une force manuelle de chaque fois 20 N appliquée de part et d'autre au prolongement d'enveloppe (64) conduise à une déformation du prolongement d'enveloppe (64), qui a pour conséquence une déformation du réservoir de fluide (20) et dès lors une opération d'expulsion.

2. Distributeur de gouttes selon la revendication 1, **caractérisé en ce que** le prolongement d'enveloppe (64) s'étend, par rapport à la direction (1) de mise en place du capuchon (60), sur au moins 75 % de la longueur (a1) du réservoir de fluide (20) entre l'unité de sortie (40) et l'extrémité opposée (26) du réservoir de fluide (20).

3. Distributeur de gouttes selon la revendication 2, **caractérisé en ce que** le prolongement d'enveloppe (64) s'étend, par rapport à la direction (1) de mise en place du capuchon (60), sur au moins 90 % de la longueur (a1) du réservoir de fluide (20), entre l'unité de sortie (40) et l'extrémité opposée (26) du réservoir de fluide (20).

4. Distributeur de gouttes selon l'une quelconque des revendications précédentes, **caractérisé en ce que** le capuchon (60) présente dans le prolongement d'enveloppe (64) au moins une, de préférence au moins deux, découpes (66) espacées en périphérie et de préférence opposées l'une à l'autre.

5. Distributeur de gouttes selon la revendication 4, **caractérisé en ce que** les découpes (66) sont profilées de telle manière que le prolongement d'enveloppe (64) soit fermé en périphérie sur au moins 50 % de la longueur (a1) du réservoir de fluide (20) entre l'unité de sortie (40) et l'extrémité opposée (26) du réservoir de fluide (20) ou que la largeur respective des découpes (66) ne dépasse pas en direction périphérique un angle de 90°.
